(19) Europäisches Patentamt
European Patent Office
Office européen des brevets

(11) EP 4 317 124 A1

(12) **EUROPEAN PATENT APPLICATION**
published in accordance with Art. 153(4) EPC

(43) Date of publication:
**07.02.2024 Bulletin 2024/06**

(21) Application number: **22775670.7**

(22) Date of filing: **23.03.2022**

(51) International Patent Classification (IPC):
***C07C 17/093*** (2006.01) ***C07C 17/361*** (2006.01)
***C07C 22/08*** (2006.01) ***C07C 41/22*** (2006.01)
***C07C 43/12*** (2006.01) ***C07C 43/192*** (2006.01)
***C07C 43/225*** (2006.01) ***C07C 231/12*** (2006.01)
***C07C 233/13*** (2006.01) ***C07C 319/20*** (2006.01)
***C07C 323/12*** (2006.01) ***C07B 39/00*** (2006.01)

(52) Cooperative Patent Classification (CPC):
**C07C 17/093; C07C 17/35; C07C 17/361; C07C 22/08; C07C 41/22; C07C 43/12; C07C 43/192; C07C 43/225; C07C 231/12; C07C 231/14; C07C 233/12; C07C 233/13; C07C 319/20; C07C 323/12;** C07B 39/00

(86) International application number:
**PCT/JP2022/013427**

(87) International publication number:
**WO 2022/202888 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2021 JP 2021052607**
**16.06.2021 JP 2021100225**

(71) Applicant: **DAIKIN INDUSTRIES, LTD.**
**Osaka-shi, Osaka 530-0001 (JP)**

(72) Inventors:
- **SHIRAI, Atsushi**
**Osaka-shi, Osaka 530-0001 (JP)**
- **KUROKI, Yoshichika**
**Osaka-shi, Osaka 530-0001 (JP)**
- **ETOU, Yuusuke**
**Osaka-shi, Osaka 530-0001 (JP)**
- **KISHIMOTO, Masayuki**
**Osaka-shi, Osaka 530-0001 (JP)**
- **NAMIKAWA, Takashi**
**Osaka-shi, Osaka 530-0001 (JP)**
- **ISHIHARA, Sumi**
**Osaka-shi, Osaka 530-0001 (JP)**
- **SUYAMA, Makoto**
**Osaka-shi, Osaka 530-0001 (JP)**
- **ADACHI, Kenji**
**Osaka-shi, Osaka 530-0001 (JP)**
- **KISHIKAWA, Yosuke**
**Osaka-shi, Osaka 530-0001 (JP)**

(74) Representative: **Hoffmann Eitle**
**Patent- und Rechtsanwälte PartmbB**
**Arabellastraße 30**
**81925 München (DE)**

# (54) METHOD FOR PRODUCING ORGANIC FLUORINE COMPOUNDS

(57) An object of the present disclosure is to provide a method for producing a fluorinated organic compound, the method being capable of removing a sulfur-containing substance to a high degree, or provide a composition containing a fluorinated organic compound with a low content of a sulfur-containing substance. The present disclosure relates to a method for producing a fluorinated organic compound ($p^f$), the method comprising:
[A] reaction step A of fluorinating an organic sulfur compound ($s^s$) with a fluorinating agent to obtain a composition ($\alpha$) containing a fluorinated organic compound ($p^f$); and
[B] post-treatment step B of reacting the composition ($\alpha$) with a nucleophile at a temperature of 40°C or higher.

EP 4 317 124 A1

## Description

Technical Field

The present disclosure relates to a method for producing a fluorinated organic compound and the like.

Background Art

Methods comprising applying a fluorinating agent, such as $IF_5$, to an organic compound containing a sulfur atom to produce a fluorinated organic compound are known (Patent Literature (PTL) 1 and Patent Literature (PTL) 2). In these methods, however, when a desired fluorinated organic compound is produced, a sulfur-containing substance, which is generated from an organic compound containing a sulfur atom (that is a starting compound) is also produced as a byproduct.

An alkali treatment method is known as a method for purifying a fluorinated organic compound (in particular, a fluorinated organic compound produced by using a fluorinating agent comprising $IF_5$) by removing this sulfur-containing substance.

Citation List

Patent Literature

PTL 1: WO2001/96263
PTL 2: WO2015/141811

Summary of Invention

Technical Problem

Although the alkali treatment method known before now can reduce the mass of a sulfur-containing substance, further reduction has been desired. An object of the present disclosure is to provide a method for producing a fluorinated organic compound, the method being capable of removing a sulfur-containing substance to a high degree, or provide a composition comprising a fluorinated organic compound with a low content of a sulfur-containing substance.

Solution to Problem

The present disclosure includes the following.

Item 1. A method for producing a fluorinated organic compound ($p^f$), comprising:

[A] a reaction step A of fluorinating an organic sulfur compound ($s^s$) with a fluorinating agent to obtain a composition ($\alpha$) containing a fluorinated organic compound ($p^f$); and
[B] a post-treatment step B of reacting the composition ($\alpha$) with a nucleophile at a temperature of 40°C or higher.

Item 2. The production method according to Item 1, wherein the reaction in the post-treatment step B is performed in the presence of water.

Item 3. The production method according to Item 1 or 2, wherein the fluorinating agent comprises a fluoride ion.

Item 4. The production method according to any one of Items 1 to 3, wherein the fluorinating agent comprises an oxidant and a fluoride ion.

Item 5. The production method according to Item 4, wherein the oxidant is at least one member selected from the group consisting of halogen oxidants and ammonium salt oxidants.

Item 6. The production method according to Item 4 or 5, wherein the oxidant comprises at least one member selected from the group consisting of

$IF_5$,

bromine trifluoride,
compounds represented by formula (3a):
$X^2$-Z (3a)

(wherein $X^2$ is a halogen atom,
Z is a halogen atom or $NZ^1Z^2$,
$Z^1$ and $Z^2$ are each independently an organic group, or
$Z^1$ and $Z^2$ bind together to form a ring), and compounds represented by formula (3a')

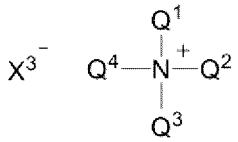
(3a')

(wherein $X^3$ is a halogen atom, $ClO_4$, or $NO_3$,
$Q^1$ to $Q^4$ are each independently H or an organic group, or any two of $Q^1$ to $Q^4$ may bind together to form a ring).

Item 7. The production method according to Item 7,
wherein

$X^2$ is an iodine atom, a bromine atom, or a chlorine atom,
Z is an iodine atom, a bromine atom, a chlorine atom, or $NZ^1Z^2$, and
$X^3$ is $ClO_4$ or $NO_3$.

Item 8. The production method according to any one of Items 1 to 6, wherein the fluorinating agent comprises $IF_5$, a fluoride ion, and an amine.

Item 9. The production method according to any one of Items 1 to 6, wherein the fluorinating agent comprises bromine trifluoride and a fluoride ion.

Item 10. The production method according to any one of Items 1 to 9, wherein the nucleophile is an anion-source compound.

Item 11. The production method according to Item 10, wherein the anion-source compound is at least one member selected from the group consisting of a carbanion-source compound, a halogen ion-source compound, and a basic compound.

Item 12. The production method according to Item 11, wherein the basic compound is an inorganic base.

Item 13. The production method according to Item 11, wherein the basic compound is a compound represented by formula:

$$M_mX_n$$

(wherein

M is a cation,
X is OH, CN, or $CO_3$,
m is the valence of X, and
n is the valence of M).

Item 14. The production method according to Item 13, wherein M is a metal cation or an optionally substituted ammonium.

Item 15. The production method according to Item 13, wherein M is an alkali metal, an alkaline earth metal, or a quaternary ammonium.

Item 16. The production method according to any one of Items 11 to 15, wherein the basic compound is one or two compounds selected from the group consisting of KOH and NaOH.

Item 17. The production method according to any one of Items 1 to 16, wherein the temperature in the post-treatment step B is 50°C or higher.

Item 18. The production method according to any one of Items 1 to 17, wherein the post-treatment step B is performed for 0.5 hours or more.

Item 19. The production method according to any one of Items 1 to 18, wherein the post-treatment step B is performed in an organic solvent WS having a solubility in water of at least 10 g/100 g of water at 20°C.

Item 20. The production method according to Item 19, wherein

the post-treatment step B is performed in a mixed solvent containing the organic solvent WS and an organic solvent WP whose solubility in water is less than 10 g/100 g of water at 20°C, and
the mass of the organic solvent WS relative to the mass of the organic solvent WP is 0.1 g/g or more.

Item 21. The production method according to any one of Items 1 to 20, wherein (1) a reaction liquid obtained by reacting the composition ($\alpha$) with a nucleophile or (2) an organic phase obtained by separating the reaction liquid is subjected to at least one treatment selected from the group consisting of crystallization, adsorption, and distillation.

Item 22. The production method according to Item 22, wherein an adsorbent used for the adsorption is silica or alumina.

Item 23. A composition ($\beta$) comprising a fluorinated organic compound ($p^f$) and a sulfur-containing substance ($b^s$) (with the proviso that the fluorinated organic compound ($p^f$) is excluded from the sulfur-containing substance ($b^s$)), wherein the content of the sulfur-containing substance ($b^s$) is 10000 ppm by mass or less in terms of sulfur.

Item 24. The composition ($\beta$) according to Item 23, wherein the content of the fluorinated organic compound ($p^f$) is 80 mass% or more.

Item 25. The composition ($\beta$) according to Item 23 or 24, wherein the mass ratio of the content of the sulfur-containing substance ($b^s$) in terms of sulfur to the content of the fluorinated organic compound ($p^f$) is 1/10 or less.

Item 26. The composition ($\beta$) according to any one of Items 23 to 25, wherein the content of the sulfur-containing substance ($b^s$) is 7000 ppm by mass or less in terms of sulfur.

Item 27. The composition ($\beta$) according to any one of Items 23 to 26, wherein the content of the sulfur-containing substance ($b^s$) is 6000 ppm by mass or less in terms of sulfur.

Item 28. The composition ($\beta$) according to any one of Items 23 to 27, wherein the content of the sulfur-containing substance ($b^s$) is 0.01 ppm by mass or more in terms of sulfur.

Item 29. The composition ($\beta$) according to any one of Items 23 to 28, wherein the content of the sulfur-containing substance ($b^s$) is 0.1 ppm by mass or more in terms of sulfur.

Advantageous Effects of Invention

According to the present disclosure, provided is a method for producing a fluorinated organic compound, the method being capable of removing a sulfur-containing substance to a high degree. Further, according to the present disclosure, provided is a composition containing a fluorinated organic compound with a low content of a sulfur-containing substance.

Description of Embodiments

The above summary of the present disclosure is not intended to describe each disclosed embodiment or every implementation of the present disclosure.
The following description of the present disclosure illustrates embodiments of examples more specifically.
In several places throughout the present disclosure, guidance is provided through lists of examples, and these examples can be used in various combinations.
In each case, the stated list of examples serves only as a representative group, and should not be interpreted as an exclusive list.
All of the publications, patents, and patent applications cited herein are incorporated herein by reference in their entirety.

Terms

The symbols and abbreviations in the present specification will be understood in the meaning usually used in the technical field of the present disclosure in the context of the present description unless otherwise specified.
The terms "comprising" and "containing" in the present specification are used with the intention of including the meaning of the phrases "consisting essentially of" and "consisting of."
The steps, treatments, or operations described in the present specification can be performed at room temperature unless otherwise specified. In the present specification, room temperature refers to a temperature in the range of 10 to 40°C.
The notation "$C_n$-$C_m$" (wherein n and m are each a number) used in the present specification means that the number of carbon atoms is n or more and m or less, as usually understood by a person skilled in the art.
Examples of the halogen atom in the present specification include a fluorine atom, a chlorine atom, a bromine atom, and an iodine atom.
The "organic group" in the present specification means a group formed by removing one hydrogen atom from an organic compound.
Examples of the "organic group" include an alkyl group optionally having one or more substituents, an alkenyl group optionally having one or more substituents, an alkynyl group optionally having one or more substituents, an aryl group optionally having one or more substituents, an aralkyl group optionally having one or more substituents, a non-aromatic heterocyclic group optionally having one or more substituents, a heteroaryl group optionally having one or more substituents, a cyano group, an aldehyde group, RO-, RCO-, RCOO-, $RSO_2$-, ROCO-, and $ROSO_2$- (wherein each R independently represents an alkyl group optionally having one or more substituents, an alkenyl group optionally having one or more substituents, an alkynyl group optionally having one or more substituents, an aryl group optionally having one or more substituents, an aralkyl group optionally having one or more substituents, a non-aromatic heterocyclic group optionally having one or more substituents, or a heteroaryl group optionally having one or more substituents).
The "alkyl group" in the present specification may be a linear alkyl group, a branched alkyl group, or a cyclic alkyl group (for example, a cycloalkyl group).
The "alkyl group" in the present specification can be, for example, a $C_1$-$C_{20}$ alkyl group, a $C_1$-$C_{12}$ alkyl group, a $C_1$-$C_6$ alkyl group, a $C_1$-$C_4$ alkyl group, or a $C_1$-$C_3$ alkyl group.
Examples of the "alkyl group" in the present specification include linear or branched alkyl groups, such as methyl, ethyl, propyl (n-propyl, isopropyl), butyl (n-butyl, isobutyl, sec-butyl, tert-butyl), pentyl, and hexyl.
Examples of the "alkyl group" in the present specification include cyclic alkyl groups or cycloalkyl groups (e.g., $C_3$-$C_8$ cycloalkyl groups) such as cyclopropyl, cyclobutyl, cyclopentyl, and cyclohexyl.
The "alkenyl group" in the present specification can be, for example, a $C_2$-$C_{10}$ alkenyl group or a $C_2$-$C_6$ alkenyl group.
Examples of the "alkenyl group" in the present specification include linear or branched alkenyl groups, such as vinyl, allyl, 3-butenyl, 1-propen-1-yl, 2-propen-1-yl, isopropenyl, 2-buten-1-yl, 4-penten-1-yl, and 5-hexen-1-yl.
Examples of the "alkenyl group" in the present specification include cyclic alkenyl groups or cycloalkenyl groups (e.g., $C_3$-$C_8$ cycloalkenyl groups), such as cyclopropenyl, cyclobutenyl, cyclopentenyl, cyclohexenyl group, and cycloheptenyl.
The "alkynyl group" in the present specification can be, for example, a $C_2$-$C_{10}$ alkynyl group.
Examples of the "alkynyl group" include linear or branched alkynyl groups, such as ethynyl, 1-propyn-1-yl, 2-propyn-1-yl, 4-pentyn-1-yl, and 5-hexyn-1-yl.
The "aryl group" in the present specification can be, for example, monocyclic, bicyclic, tricyclic, or tetracyclic.
The "aryl group" in the present specification can be, for example, $C_6$-$C_{28}$ aryl, $C_6$-$C_{16}$ aryl, $C_6$-$C_{14}$ aryl, or $C_6$-$C_{12}$ aryl.
Examples of the "aryl group" in the present specification include phenyl, 1-naphthyl, 2-naphthyl, 2-biphenyl, 3-biphenyl, 4-biphenyl, and 2-anthryl.

The "aryloxy group" in the present specification can be a group in which an oxygen atom is bonded to an aryl group (aryl group-O-).

The "aryloxy group" in the present specification can be, for example, $C_6$-$C_{18}$ aryloxy, $C_6$-$C_{16}$ aryloxy, $C_6$-$C_{14}$ aryloxy, or $C_6$-$C_{12}$ aryloxy.

Examples of the "aryloxy group" in the present specification include phenoxy and naphthyloxy.

Examples of the "aralkyl group" in the present specification include $C_7$-$C_{19}$ aralkyl, $C_7$-$C_{17}$ aralkyl, $C_7$-$C_{15}$ aralkyl, $C_7$-$C_{13}$ aralkyl, and $C_7$-$C_{10}$ aralkyl.

Examples of the "aralkyl group" in the present specification include benzyl, phenethyl, diphenylmethyl, 1-phenylethyl, 1-naphthylmethyl, 2-naphthylmethyl, 2,2-diphenylethyl, 3-phenylpropyl, 4-phenylbutyl, 5-phenylpentyl, 2-biphenylmethyl, 3-biphenylmethyl group, and 4-biphenylmethyl group.

The "non-aromatic heterocyclic group" in the present specification can be monocyclic, bicyclic, tricyclic, or tetracyclic.

The "non-aromatic heterocyclic group" in the present specification includes a non-aromatic heterocyclic group containing, in addition to carbon atoms, 1 to 4 heteroatoms selected from oxygen, sulfur, and nitrogen atoms as ring-constituting atoms.

The "non-aromatic heterocyclic group" in the present specification can be saturated or unsaturated.

Examples of the "non-aromatic heterocyclic group" in the present specification include tetrahydrofuryl, oxazolidinyl, imidazolinyl, aziridinyl, azetidinyl, pyrrolidinyl, piperidinyl, azepanyl, azocanyl, piperazinyl, diazepinyl, diazocanyl, tetrahydropyranyl, morpholinyl, thiomorpholinyl, 2-oxazolidinyl, dihydrofuryl, dihydropyranyl, and dihydroquinolyl.

Examples of the "heteroaryl group" in the present specification include monocyclic aromatic heterocyclic groups (e.g., 5- or 6-membered monocyclic aromatic heterocyclic groups) and aromatic condensed heterocyclic groups (e.g., 5- to 18-membered aromatic condensed heterocyclic groups).

Examples of the "5- or 6-membered monocyclic aromatic heterocyclic groups" in the present specification include pyrrolyl, furyl, thienyl, pyrazolyl, imidazolyl, isoxazolyl, oxazolyl, isothiazolyl, thiazolyl, triazolyl, oxadiazolyl, thiadiazolyl, tetrazolyl, pyridyl, pyridazinyl, pyrimidinyl, and pyrazinyl.

Examples of the "5- to 18-membered aromatic fused heterocyclic group" in the present specification include isoindolyl, indolyl, benzofuranyl, benzothienyl, indazolyl, benzoimidazolyl, 1,2-benzoisoxazolyl, benzoxazolyl, 1,2-benzoisothiazolyl, benzothiazolyl, isoquinolyl, quinolyl, cinnolinyl, phthalazinyl, quinazolinyl, quinoxalinyl, pyrazolo[1,5-a]pyridyl, and imidazo[1,2-a]pyridyl.

Examples of "RO-" in the present specification include alkoxy groups, aryloxy groups (e.g., $C_6$-$C_{18}$ aryloxy groups, such as phenoxy and naphthoxy), and aralkyloxy groups (e.g., $C_7$-$C_{19}$ aralkyloxy groups, such as benzyloxy and phenethyloxy).

The "alkoxy group" in the present specification can be a group to which an oxygen atom is bound to alkyl (alkyl-O-).

Examples of the "alkoxy group" in the present specification include linear or branched alkoxy groups, such as $C_1$-$C_{20}$ alkoxy groups, $C_1$-$C_{12}$ alkoxy groups, $C_1$-$C_6$ alkoxy groups, $C_1$-$C_4$ alkoxy groups, and $C_1$-$C_3$ alkoxy groups; and cyclic alkoxy groups, such as $C_3$-$C_8$ cycloalkoxy groups, $C_3$-$C_7$ cycloalkoxy groups, and $C_4$-$C_7$ cycloalkyl groups.

Examples of the "alkoxy group" in the present specification include linear or branched alkoxy groups, such as methoxy, ethoxy, propoxy (n-propoxy, isopropoxy), butoxy (n-butoxy, isobutoxy, sec-butoxy, and tert-butoxy), pentyloxy, and hexyloxy; and cyclic alkoxy groups, such as cyclopropoxy, cyclobutoxy, cyclopentoxy, and cyclohexoxy.

Examples of "RCO-" in the present specification include alkylcarbonyl groups (e.g., ($C_1$-$C_{10}$ alkyl)carbonyl groups, such as acetyl, propionyl, and butyryl), arylcarbonyl groups (e.g., ($C_6$-$C_{18}$ aryl)carbonyl groups, such as benzoyl and naphthoyl), and aralkylcarbonyl groups (e.g., ($C_7$-$C_{19}$ aralkyl)carbonyl groups, such as benzylcarbonyl and phenethylcarbonyl) .

Examples of the "RCOO-" in the present specification include alkylcarbonyloxy groups (e.g., ($C_1$-$C_{10}$ alkyl)carbonyloxy groups, such as acetyloxy, propionyloxy, and butyryloxy), arylcarbonyloxy groups (e.g., ($C_6$-$C_{18}$ aryl)carbonyloxy groups, such as benzoyloxy and naphthoyloxy), and aralkylcarbonyloxy groups (e.g., ($C_7$-$C_{19}$ aralkyl)carbonyloxy groups, such as benzylcarbonyloxy and phenethylcarbonyloxy) .

Examples of the "$RSO_2$-" in the present specification include alkylsulfonyl groups (e.g., $C_1$-$C_{10}$ alkylsulfonyl groups, such as methylsulfonyl, ethylsulfonyl, and propylsulfonyl), arylsulfonyl groups (e.g., $C_6$-$C_{18}$ arylsulfonyl groups, such as phenylsulfonyl and naphthylsulfonyl), and aralkylsulfonyl groups (e.g., $C_7$-$C_{19}$ aralkylsulfonyl groups, such as benzylsulfonyl and phenethylsulfonyl) .

Examples of the "ROCO-" in the present specification include alkoxycarbonyl groups (e.g., ($C_1$-$C_{10}$ alkoxy)carbonyl groups, such as methoxycarbonyl, ethoxycarbonyl, and propoxycarbonyl), aryloxycarbonyl groups (e.g., ($C_6$-$C_{18}$ aryloxy)carbonyl groups, such as phenoxycarbonyl and naphthoxycarbonyl), and aralkyloxycarbonyl groups (e.g., ($C_7$-$C_{19}$ aralkyloxy)carbonyl groups, such as benzyloxycarbonyl and phenethyloxycarbonyl).

Examples of the "$ROSO_2$-" in the present specification include alkoxysulfonyl groups (e.g., $C_1$-$C_{10}$ alkoxysulfonyl

groups, such as methoxysulfonyl, ethoxysulfonyl, and propoxysulfonyl), aryloxysulfonyl groups (e.g., $C_6$-$C_{18}$ aryloxysulfonyl groups, such as phenoxysulfonyl and naphthoxysulfonyl), and aralkyloxysulfonyl groups (e.g., $C_7$-$C_{19}$ aralkyloxysulfonyl groups, such as benzyloxysulfonyl and phenethyloxysulfonyl).

Examples of substituents in the "alkyl group optionally having one or more substituents," "alkenyl group optionally having one or more substituents," "alkynyl group optionally having one or more substituents," "aryl group optionally having one or more substituents," "aralkyl group optionally having one or more substituents," "non-aromatic heterocyclic group optionally having one or more substituents," and "heteroaryl group optionally having one or more substituents" in the present specification include a halo group, a nitro group, a cyano group, an oxo group, a thioxo group, a carboxyl group, a sulfo group, a sulfamoyl group, a sulfinamoyl group, a sulfenamoyl group, RO-, RCO-, RCOO-, $RSO_2$-, ROCO-, and $ROSO_2$- (in these formulas, $R^r$ is as defined above).

Of these substituents, examples of the "halo group" can include fluoro, chloro, bromo, and iodo groups.

The number of substituents can be within the range of 1 to the maximum substitutable number (e.g., 1, 2, 3, 4, 5, or 6).

Examples of the "heterocycloalkyl group" in the present specification include groups in which one or more carbon atoms that constitute the ring structure of the cycloalkyl groups described above are substituted with other atoms, such as nitrogen, oxygen, or sulfur.

Examples of the "monoalkylamino group" in the present specification include an amino group mono-substituted with a $C_1$-$C_6$ alkyl group.

Examples of the "dialkylamino group" in the present specification include an amino group di-substituted with $C_1$-$C_6$ alkyl groups that are the same or different.

Examples of the dialkylamino group include amino disubstituted with $C_1$-$C_6$ alkyl groups, such as dimethylamino, diethylamino, di-n-propylamino, diisopropylamino, dibutylamino, dipentylamino, dihexylamino, methylethylamino, methylpropylamino, methylbutylamino, methylpentylamino, methylhexylamino, ethylpropylamino, ethylbutylamino, ethylpentylamino, ethylhexylamino, propylbutylamino, propylpentylamino, propylhexylamino, butylpentylamino, butylhexylamino, and pentylhexylamino.

Examples of the "acylamino group" include acylamino groups having 1 to 8 carbon atoms, such as formylamino, benzoylamino, acetylamino, propionylamino, and n-butyrylamino (e.g., formylamino, alkanoylamino, and arylcarbonylamino).

Examples of the "alkylthio group" in the present specification include -S-($C_1$-$C_6$ alkyl) (wherein the $C_1$-$C_6$ alkyl group is as defined above).

Examples of heterocyclic groups in the present specification include 5- to 10-membered monocyclic or bicyclic heterocyclic groups having at least one heteroatom selected from the group consisting of nitrogen, oxygen, and sulfur as a ring-constituting atom, such as piperidyl, furyl, thienyl, imidazolyl, oxazolyl, thiazolyl, pyrrolyl, pyrrolidinyl, triazolyl, benzothiazolyl, benzimidazolyl, oxadiazolyl, thiadiazolyl, indolyl, pyrazolyl, pyridazinyl, cinnolinyl, quinolyl, isoquinolyl, quinoxalinyl, pyrazinyl, pyridyl, benzofuryl, benzothienyl, and tetrazolyl.

Among the heterocyclic groups, examples of the "aromatic heterocyclic group" include 5- to 10-membered monocyclic or bicyclic heteroaryl groups having at least one heteroatom selected from nitrogen, oxygen, and sulfur as a ring-constituting atom, such as furyl, thienyl, imidazolyl, oxazolyl, thiazolyl, pyrrolyl, triazolyl, benzothiazolyl, benzimidazolyl, oxadiazolyl, thiadiazolyl, indolyl, pyrazolyl, pyridazinyl, cinnolinyl, quinolinyl, isoquinolinyl, quinoxalinyl, pyrazinyl, pyridyl, benzofuryl, benzothienyl, and tetrazolyl.

Examples of the "acyl group" in the present specification include formyl; linear or branched $C_2$-$C_6$ alkanoyl groups, such as acetyl, propionyl, n-butyryl, isobutyryl, valeryl, isovaleryl, and pivaloyl; and $C_7$-$C_{15}$ arylcarbonyl groups, such as benzoyl.

Examples of the alkyl, aralkyl, aryl, cycloalkyl, heterocycloalkyl, and heterocyclic groups of the "alkylsulfinyl group," "aralkylsulfinyl group," "arylsulfinyl group," "cycloalkylsulfinyl group," "heterocycloalkylsulfinyl group," and "sulfinyl group having a heterocyclic group bound thereto" in the present specification include those mentioned above.

Examples of the alkyl, aralkyl, aryl, cycloalkyl, heterocycloalkyl, and heterocyclic groups of the "alkylsulfonyl group," "aralkylsulfonyl group," "arylsulfonyl group," "cycloalkylsulfonyl group," "heterocycloalkylsulfonyl group," and "sulfonyl group having a heterocyclic group bound thereto" in the present specification include those mentioned above.

Examples of the "ester group" in the present specification include acyl-O- and alkoxy-CO-. Examples of the "acyl" and "alkoxy" include "acyl groups" and "alkoxy groups" described above.

The number of substituents in the "alkyl group having one or more substituents," "alkoxy group having one or more substituents," or "alkenyl group having one or more substituents" in the present specification can be within the range of 1 to the maximum substitutable number, for example, 1 to 10, 1 to 8, 1 to 6, or 1 to 5, and preferably 1 to 3. The substituent is not particularly limited as long as it is a substituent that is inactive in the fluorination reaction with a fluorinating agent described below. Examples of substituents include halogen atoms, $C_1$-$C_6$ alkoxy groups, $C_1$-$C_6$ alkylthio groups, a cyano group, a nitro group, an amino group, a hydroxyl group, $C_1$-$C_6$ alkyl-carbonyloxy groups (e.g., acetoxy), $C_1$-$C_6$ alkoxy-carbonyl groups (e.g., isopropyloxycarbonyl), and $C_3$-$C_6$ cycloalkyl groups (e.g., cyclohexyl). Examples of

the alkyl group having a halogen atom include alkyl groups in which a part or all of the hydrogen atoms are substituted with fluorine.

The number of substituents in the "aralkyl group having one or more substituents," "aryl group having one or more substituents," "aryloxy group having one or more substituents," "cycloalkyl group having one or more substituents," "heterocycloalkyl group having one or more substituents," "heterocyclic group having one or more substituents", "monoalkylamino group having one or more substituents," "dialkylamino group having one or more substituents," "acylamino group having one or more substituents," "alkylsulfinyl group having one or more substituents," "aralkylsulfinyl group having one or more substituents," "arylsulfinyl group having one or more substituents," "cycloalkylsulfinyl group having one or more substituents", "heterocycloalkylsulfinyl group having one or more substituents," "sulfinyl group to which a heterocyclic group having one or more substituents is bonded," "alkylsulfonyl group having one or more substituents," "aralkylsulfonyl group having one or more substituents," "arylsulfonyl group having one or more substituents," "cycloalkylsulfonyl group having one or more substituents," "heterocycloalkylsulfonyl group having one or more substituents," and "sulfonyl group to which a heterocyclic group having one or more substituents is bonded" in the present specification is, for example, 1 to 5, and preferably 1 to 3. The substituent is not particularly limited as long as it is a substituent that is inactive in the fluorination reaction with a fluorinating agent described below. Examples of the substituent include $C_1$-$C_6$ alkyl groups; halogen atoms; $C_1$-$C_6$ alkoxy groups; $C_1$-$C_6$ alkylthio groups; a cyano group; a nitro group; an amino group; a hydroxyl group; an oxo group (=O); a cyclohexyl group optionally substituted with a cycloalkyl group optionally substituted with 1 to 3 (e.g., 1 or 2) $C_1$-$C_{10}$ alkyl groups (e.g., $C_1$-$C_6$ alkyl groups); a phenyl group optionally substituted with 1 to 3 (e.g., 1 or 2) substituents (e.g., alkyl (e.g., $C_1$-$C_{10}$ alkyl or $C_1$-$C_6$ alkyl) and halogen atoms (e.g., a fluoro atom or a chloro atom)); and a biphenyl group optionally substituted with 1 to 3 (e.g., 1 or 2) substituents (e.g., alkyl groups (e.g., $C_1$-$C_{10}$ alkyl or $C_1$-$C_6$ alkyl) and halogen atoms (e.g., a fluorine atom or a chlorine atom)).

The number of "substituents in the "4- to 7-membered aliphatic ring having one or more substituents" in the present specification may be, for example, 1 to 5, and preferably 1 to 3. The substituent is not particularly limited as long as it is a substituent that is inactive in the fluorination reaction with a fluorinating agent described below. Examples of substituents include $C_1$-$C_6$ alkyl groups, halogen atoms, $C_1$-$C_6$ alkoxy groups, $C_1$-$C_6$ alkylthio groups, a cyano group, a nitro group, an amino group, a hydroxyl group, and carboxy esters.

Further, the compound represented by the following formula:

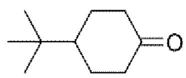

is also included in the scope of the aliphatic 4- to 7-membered ring having one or more substituent.

The number of substituents in the "acyl group having one or more substituents" in the present specification is, for example, 1 to 5, and preferably 1 to 3. The substituent is not particularly limited as long as it is a substituent that is inactive in the fluorination reaction with a fluorinating agent described below. Examples of substituents include halogen-substituted acetyl groups, such as chloroacetyl, bromoacetyl, dichloroacetyl, and trifluoroacetyl; alkoxy-substituted acetyl groups, such as methoxyacetyl and ethoxyacetyl; alkylthio-substituted acetyl groups, such as methylthioacetyl; phenoxyacetyl; phenylthioacetyl; and substituted benzoyl groups, such as 2-chlorobenzoyl, 3-chlorobenzoyl, 4-chlorobenzoyl, 4-methylbenzoyl, 4-t-butylbenzoyl, 4-methoxybenzoyl, 4-cyanobenzoyl, and 4-nitrobenzoyl.

## Method for producing fluorinated organic compound

In one embodiment, the method for producing a fluorinated organic compound ($p^f$) comprises:

[A] a reaction step A of fluorinating an organic sulfur compound ($s^s$) with a fluorinating agent to obtain a composition ($\alpha$) containing a fluorinated organic compound ($p^f$); and

[B] a post-treatment step B of reacting the composition ($\alpha$) with a nucleophile at a temperature of 40°C or higher.

## Reaction step A

The composition ($\alpha$) is not particularly limited as long as the composition contains a fluorinated organic compound ($p^f$) produced from an organic sulfur compound ($s^s$) by using a fluorinating agent, the fluorinated organic compound ($p^f$) corresponding to the organic sulfur compound ($s^s$). The composition ($\alpha$) can be, for example, a crude fluorinated organic compound containing a sulfur-containing substance ($b^s$) as an impurity. The fluorinated organic compound ($p^f$) is excluded from the scope of the sulfur-containing substance ($b^s$).

Examples of the fluorination in the production method according to the present disclosure are described below. The examples of the fluorinated organic compound ($p^f$) obtained by the production method of the present disclosure can

also be thereby provided.

Examples of the organic sulfur compound ($s^s$) include sulfide compounds (thioether compounds) and thiocarbonyl compounds.

In the sulfide compound (including dithioacetal and dithioketal), for example, one or two hydrogen atoms of methylene adjacent to the sulfur atom are replaced with one or two fluorine atoms, or the sulfur atom is replaced with a fluorine atom to form an organic sulfur compound ($s^s$):

(a-1) $R^3$-$CH_2$-S-$R^{3a}$ → $R^3$-CFH-S-$R^{3a}$ -> $R^3$-$CF_2$-S-$R^{3a}$
(a-2) $R^3$-$CHR^{3b}$-S-$R^{3a}$ → $R^3$-$CFR^{3b}$-S-$R^{3a}$
(b-1) $R^3$-CO-$CH_2$-S-$R^{3a}$ → $R^3$-CO-CFH-S-$R^{3a}$ → $R^3$-CO-$CF^2$-S-$R^{3a}$
(b-2) $R^3$-CO-$CHR^{3b}$-S-$R^{3a}$ → $R^3$-CO-$CFR^{3b}$-S-$R^{3a}$
(c) $R^{3c}R^{3d}C=C(SR^{3a})_2$ → $R^{3c}R^{3d}CH$-$CF_2$-$SR^{3a}$
(d) $R^{3c}R^{3d}C(SR^{3a'})(SR^{3a''})$ → $R^{3c}R^{3d}CF_2$
(e) $R^3$-$C(SR^{3a})(SR^{3a'})(SR^{3a''})$ → $R^3$-$CF_3$
(f) $R^3$-$C(SR^{3a})(SR^{3a'})$-S-$R^{3e}$-S-$(SR^{3a'})(SR^{3a})$-$R^3$ → $R^3$-$CF_3$

(wherein $R^{3a}$, $R^{3a'}$, and $R^{3a''}$ are the same or different, and each represent an alkyl group optionally having one or more substituents, an aralkyl group optionally having one or more substituents, an aryl group optionally having one or more substituents, an alkenyl group optionally having one or more substituents, a cycloalkyl group optionally having one or more substituents, a heterocycloalkyl group optionally having one or more substituents, or a heterocyclic group optionally having one or more substituents, or

$R^3$a and $R^{3a'}$ bind together to form a 4- to 7-membered aliphatic ring optionally having one or more substituents;
$R^3$ and $R^{3b}$ are the same or different, and each represent an alkyl group optionally having one or more substituents, an aralkyl group optionally having one or more substituents, an aryl group optionally having one or more substituents, an alkenyl group optionally having one or more substituents, a cycloalkyl group optionally having one or more substituents, a heterocycloalkyl group optionally having one or more substituents, a heterocyclic group optionally having one or more substituents, an alkoxy group optionally having one or more substituents, an aryloxy group optionally having one or more substituents, an amino group, a monoalkylamino group optionally having one or more substituents, a dialkylamino group optionally having one or more substituents, an acyl group, an acylamino group, a cyano group, an alkylsulfinyl group optionally having one or more substituents, an aralkylsulfinyl group optionally having one or more substituents, an arylsulfinyl group optionally having one or more substituents, a cycloalkylsulfinyl group optionally having one or more substituents, a heterocycloalkylsulfinyl group optionally having one or more substituents, a sulfonyl group to which a heterocyclic group optionally having one or more substituents is bonded, an alkylsulfonyl group optionally having one or more substituents, an aralkylsulfonyl group optionally having one or more substituents, an arylsulfonyl group optionally having one or more substituents, a cycloalkylsulfonyl group optionally having one or more substituents, a heterocycloalkylsulfonyl group optionally having one or more substituents, or a sulfonyl group to which a heterocyclic group optionally having one or more substituents is bonded, or $R^3$ and $R^{3b}$, taken together with the carbon atom to which they are attached, optionally form a 4- to 8-membered ring via or not via a heteroatom (the ring is optionally substituted with one or more substituents selected from the group consisting of a halogen atom, an oxo group, or an alkyl group optionally having one or more substituents, an aralkyl group optionally having one or more substituents, an aryl group optionally having one or more substituents, an alkenyl group optionally having one or more substituents, a cyano group, and an amino group);
wherein $R^{3c}$ and $R^{3d}$ are the same or different, and each represent a hydrogen atom, an alkyl group optionally having one or more substituents, an aralkyl group optionally having one or more substituents, an aryl group optionally having one or more substituents, an alkenyl group optionally having one or more substituents, a cycloalkyl group optionally having one or more substituents, a heterocycloalkyl group optionally having one or more substituents, a heterocyclic group optionally having one or more substituents, an alkoxy group optionally having one or more substituents, an aryloxy group optionally having one or more substituents, a monoalkylamino group optionally having one or more substituents, a dialkylamino group optionally having one or more substituents, an acyl group, or an acylamino group; or $R^{3c}$ and $R^{3d}$, taken together with the adjacent carbon atom, optionally form a saturated or unsaturated 4- to 7-membered aliphatic ring optionally having one or more substituents (the ring may be substituted with at least one member selected from the group consisting of a halogen atom, an oxo group, an alkyl group optionally having one or more substituents, an aralkyl group optionally having one or more substituents, an aryl group optionally having one or more substituents, an alkenyl group optionally having one or more substituents, a cyano group, and an amino group); and $R^{3e}$ represents an alkylene or arylene group.)

Examples of sulfide compounds include methyl ethyl sulfide, methyl benzyl sulfide, 2-phenylthioacetate, 2-

phenylthioacetophenone, $C_6H_5$-CO-$CH_2SCH_3$, bis(methylthio)methylbenzene, 2-octyl-1,3-dithiane, 2-phenyl-2-trifluoromethyl-1,3-dithiolane, tris(ethylthio)hexane, 4-tris(methylthio)toluene, 2,2-diphenyl-1,3-dithiolane, and compounds represented by the following formulas:

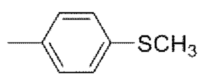
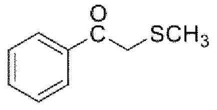
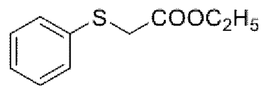
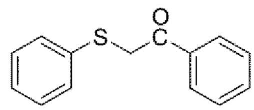

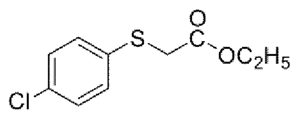
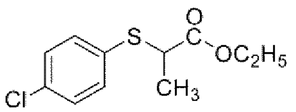
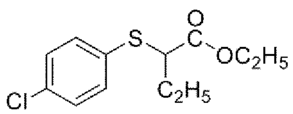

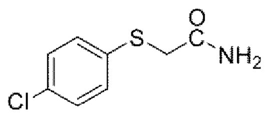
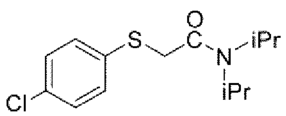
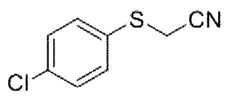

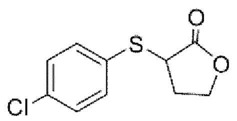
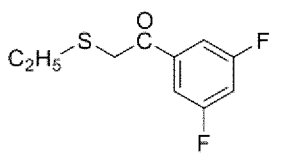
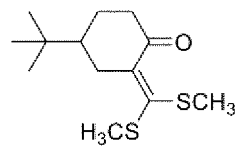

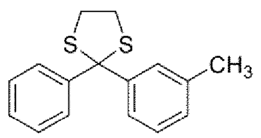
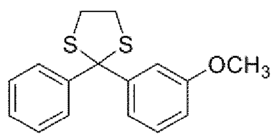
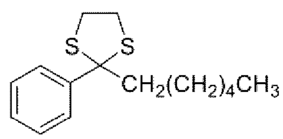
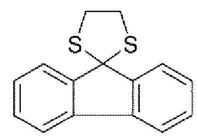

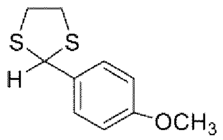
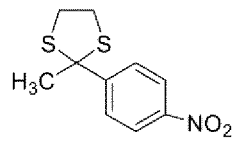
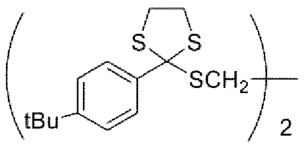
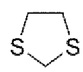

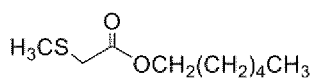
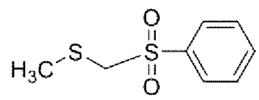

When a thiocarbonyl compound (including thioketone, thioester, thiocarbonic ester, thioamide, dithiocarboxylate, and dithiocarbamate) is used, for example, the following reaction is performed:

(a) $R^6$-C(=S) -$R^6$a → $R^6$-CF2-$R^6$a
(b) $R^6$-C(=S) -S$R^6$b → $R^6$-CF2-S$R^6$a → $R^6$-$CF_3$

(wherein $R^6$ and $R^6$a are the same or different, and each represent a hydrogen atom, an alkyl group optionally having one or more substituents, an aralkyl group optionally having one or more substituents, an aryl group optionally having one or more substituents, an alkenyl group optionally having one or more substituents, a cycloalkyl group optionally having one or more substituents, a heterocycloalkyl group optionally having one or more substituents, a heterocyclic group optionally having one or more substituents, an alkoxy group optionally having one or more substituents, an aryloxy group optionally having one or more substituents, an amino group, a monoalkylamino group optionally having one or more substituents, a dialkylamino group optionally having one or more substituents, an acyl group, or an acylamino group;

$R^6$ and $R^{6a}$ may bind together to form a ring structure;

each $R^{6a}$ may be may be the same or different, and represents a hydrogen atom, an alkyl group optionally having one or more substituents, an aralkyl group optionally having one or more substituents, an aryl group optionally having one or more substituents, an alkenyl group optionally having one or more substituents, a cycloalkyl group optionally having one or more substituents, a heterocycloalkyl group optionally having one or more substituents, a heterocyclic group optionally having one or more substituents, an alkoxy group optionally having one or more substituents, an aryloxy group optionally having one or more substituents, an amino group, a monoalkylamino group optionally having one or more substituents, a dialkylamino group optionally having one or more substituents, an acyl group optionally having one or more substituents, or an acylamino group optionally having one or more substituents;

$R^{6a}$ and $R^{6b}$ may bind together to form a ring structure;

$R^{6b}$ represents an alkyl group optionally having one or more substituents, an aralkyl group optionally having one or more substituents, an aryl group optionally having one or more substituents, an alkenyl group optionally having one or more substituents, a cycloalkyl group optionally having one or more substituents, a heterocycloalkyl group optionally having one or more substituents, or a heterocyclic group optionally having one or more substituents).

Examples of thiocarbonyl compounds include thiobenzophenone, O-decyl S-methyl dithiocarbonate, O-(4'-pentyl-[1,1'-bi(cyclohexane)]-4-yl)S-methyl dithiocarbonate, N-butyl-N-(methylthio(thiocarbonyl))propanamide, O-(3,4,5-trifluorophenyl)4-chloro-2,6-difluorobenzothioate, 0-(3,4,5-trifluorophenyl)2',3,5-trifluoro-4"-propyl-[1,1':4',1"-terphenyl]-4-carbothioate, O-(3,4,5-trifluorophenyl)2',3,5-trifluoro-4"-pentyl-[1,1':4',1"-terphenyl]-4-carbothioate, O-(4-isopropylphenyl)S-methyl dithiocarbonate, O-(4-bromophenyl)S-methyl dithiocarbonate, ethyl 4-(((methylthio)carbonothioyl)oxy)benzoate, O-(3-phenylpropyl)S-methyl dithiocarbonate, O-methyl cyclohexanecarbothioate, O-propyl 1-piperidinecarbothioate, methyl dithiobenzoate, O-phenyl thiobenzoate, N,N-dimethylphenylthioamide, ethyl 3-quinolinedithiocarboxylate, trifluoromethanecarbothioylnaphthalene, N-methyl-N-phenyltrifluoromethanethioamide, and N-benzyl-N-phenylheptafluoropropanethioamide.

The fluorinating agent can be, for example, a fluorinating agent containing a fluoride ion. The fluorinating agent containing a fluoride ion is preferably a fluorinating agent comprising an oxidant and a fluoride ion, and more preferably a fluorinating agent comprising at least one oxidant selected from the group consisting of $IF_5$, bromine trifluoride, compounds represented by the following formula (3a), and compounds represented by the following formula (3a'), and a fluoride ion. The fluorinating agent containing $IF_5$ and a fluoride ion is preferably a fluorinating agent comprising $IF_5$, a fluoride ion (preferably HF), and an amine.

$X^2$-Z (3a)

(wherein $X^2$ is a halogen atom,

Z is a halogen atom or $NZ^1Z^2$,

$Z^1$ and $Z^2$ each independently represent an organic group, or $Z^1$ and $Z^2$ may bind together to form a ring structure). formula (3a'):

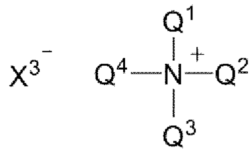

(3a')

(wherein

$X^3$ is a halogen atom, $ClO_4$, or $NO_3$;

$Q^1$ to $Q^4$ each independently represent H or an organic group; and any two of $Q^1$ to $Q^4$ may bind together to form a ring).

The oxidant is preferably a halogen oxidant or an ammonium salt oxidant. Oxidants may be used alone or in a combination of two or more. When the oxidant contains both a halogen atom and an ammonium cation, the oxidant is classified as a halogen oxidant.

The halogen oxidant may be an oxidant containing a halogen atom. Examples of the halogen oxidant include $IF_5$, bromine trifluoride, compounds represented by formula (3a), and compounds represented by formula (3a') and containing a halogen atom. The halogen oxidant is preferably $IF_5$, bromine trifluoride, or a compound represented by formula (3a).

$X^2$ is preferably an iodine atom, a bromine atom, or a chlorine atom, and more preferably a bromine atom.

Z is preferably an iodine atom, a bromine atom, a chlorine atom, or $NZ^1Z^2$.

$Z^1$ and $Z^2$ each independently represent an organic group, or $Z^1$ and $Z^2$ bind together to form a ring. Examples of the organic group include alkyl groups optionally having one or more substituents and acyl groups optionally having one or more substituents. The organic group is preferably alkylcarbonyl or arylcarbonyl.

The ring formed by bonding $Z^1$ and $Z^2$ together may have one or more substituents. Examples of the ring include a pyrrolidine ring, an imidazolidine ring, an oxopyrrolidine ring, a pyridine ring, a piperidine ring, an isoquinoline ring, a quinoline ring, a pyrazole ring, an imidazole ring, an imidazoline ring, a benzimidazole ring, a triazole ring, and a hydantoin ring. The ring is preferably a pyrrolidine ring, an imidazolidine ring, or an oxopyrrolidine ring. Examples of substituents of the ring include an oxo group and $C_1$-$C_{10}$ alkyl groups (preferably $C_1$-$C_4$ alkyl groups). Preferable examples of substituents are an oxo group and $C_1$-$C_3$ alkyl groups. The number of substituents can be, for example, 0 to 4, 0 to 3, 0 to 2, 1, 0, or 2 to 4.

Examples of the compound represented by formula (3a) include N-bromosuccinimide (which may be referred to as "NBS"), 1,3-dibromo-5,5-dimethylhydantoin (which may be referred to as "DBH"), ClF, BrF, ICl, and IBr. Preferable are, for example, N-bromosuccinimide and 1,3-dibromo-5,5-dimethylhydantoin.

$X^3$ is preferably an iodine atom, a bromine atom, $ClO_4$, or $NO_3$, and ismore preferably $ClO_4$ or $NO_3$.

$Q^1$ to $Q^4$ are each independently H or an organic group, and any two of $Q^1$ to $Q^4$ may bind together to form a ring. Examples of the organic group include alkyl groups, alkenyl groups, aryl groups, aralkyl groups, and cycloalkyl groups. These groups may have one or more substituents. The organic group is preferably an alkyl group optionally having one or more substituents, an alkenyl group optionally having one or more substituents, and an aryl group optionally having one or more substituents.

The ring formed by bonding any two of $Q^1$ to $Q^4$ together may have one or more substituents. Examples of the ring include a pyridine ring, a quinolium ring, an isoquinolium ring, a pyrrolidine ring, an imidazolidine ring, a pyrazole ring, and an imidazole ring. Preferable are, for example, a pyridine ring, an imidazolidine ring, and a pyrazole ring. Examples of the substituent of the ring include alkyl groups, alkenyl groups, aryl groups, aralkyl groups, and cycloalkyl groups. The number of substituents may be, for example, 0 to 4, 0 to 3, 0 to 2, 1, or 0, and is preferably 0 to 2.

More preferably, $Q^1$ to $Q^4$ each independently represent H or $C_1$-$C_4$ alkyl optionally substituted with one phenyl group.

Examples of the compound represented by formula (3a') include halogen oxidants, such as $NH_4Br$, $NH_4I$, $MeNH_3Br$, $MeNH_3I$, $Me_4NBr$, $Me_4NI$, $Et_4NBr$, $Et_4NI$, $Bu_4NBr$, $Bu_4NI$, $PhMe_3NBr$, $PhMe_3NI$, $PhCH_2NMe_3I$, and $NH_4ClO_4$, and ammonium oxidants, such as $NH_4NO_3$. $NH_4ClO_4$ and $NH_4NO_3$ preferable.

The fluoride ion can be a compound containing a fluoride ion. Examples include HF, KF, $KHF_2$, NaF, CsF, $TiF_4$, LiF, $MgF_2$, $CaF_2$, $NH_4F$, $NHMe_3F$, $NHEt_3F$, $NMe_4F$, $NEt_4F$, $NHBu_3F$, and $NBu_4F$. Preferable examples include HF and $KHF_2$.

Examples of the amine include primary amines, such as methyl amine, ethyl amine, propyl amine, butyl amine, pentyl amine, hexyl amine, cyclohexyl amine, and ethylene diamine; secondary amines, such as dimethyl amine, diethyl amine, dipropyl amine, dibutyl amine, dipentyl amine, dihexyl amine, and dicyclohexyl amine; and tertiary amines, such as trimethylamine, triethyl amine (which may also be indicated as "$Et_3N$"), diisopropylethylamine, tributylamine, N,N,N',N'-tetramethylethylenediamine, triphenylamine, and diphenylmethylamine; cycloaliphatic amines, such as piperidine, piperazine, pyrrolidine, morpholine, N-methylpiperazine, N-methylpyrrolidine, 5-diazabicyclo[4.3.0]nonan-5-ene, and 1,4-diazabicyclo[2.2.2]octane; aromatic amines, such as aniline, methylaniline, dimethylaniline, N,N-dimethylaniline, haloaniline, and nitroaniline; and heterocyclic amines, such as pyridine, pyromidine, piperazine, quinoline, and imidazole.

Examples of the fluorinating agent include $IF_5$-$Et_3N$-3HF, $IF_5$-$Et_3N$-5HF, $IF_5$-pyridine-HF, NBS (N-bromosuccinimide) - pyridine-9HF, DBH (1,3-dibromo-5,5-dimethylhydantoin)-pyridine-9HF, $BrF_3$-$KHF_2$, 4-tert-butyl-2,6-dimethylphenylsulfur trifluoride (e.g., FLUOLEAD (registered trademark), $Et_2NSF_3$ (abbreviated name: DAST), $(MeOCH_2CH_2)_2NSF_3$ (abbreviated name: Deoxo-Fluor), and 1-chloromethyl-4-fluoro-1,4-diazoniabicyclo[2.2.2]octanebis(tetrafluoroborate) (such as Selectfluor (registered trademark)); and preferably $IF_5$-$Et_3N$-3HF, $IF_5$-$Et_3N$-5HF, $IF_5$-pyridine-HF, NBS-pyridine-9HF, DBH-pyridine-9HF, and $BrF_3$-$KHF_2$.

The amount of the fluorinating agent used in reaction step A is preferably in the range of 0.01 to 20 moles, more preferably in the range of 0.1 to 10 moles, and still more preferably in the range of 0.1 to 5 moles, per mole of the organic sulfur compound ($s^s$) as the starting compound.

The reaction temperature in reaction step A may be usually about -70 to 200°C, preferably about -20 to 120°C, and more preferably about 0 to 100°C.

The reaction time in reaction step A is usually in the range of 0.1 hours to 300 hours, preferably in the range of 0.5 hours to 100 hours, and more preferably in the range of 2 hours to 48 hours.

Reaction step A can be carried out in the presence or absence of a solvent, and can be preferably carried out in the presence of a solvent.

Examples of the solvent include aliphatic solvents, such as pentane, hexane, heptane, cyclohexane, and petroleum ether; halogenated aliphatic solvents, such as dichloromethane, dichloroethane, chloroform, fluorotrichloromethane, 1,1,2-trichlorotrifluoroethane, 2-chloro-1,2-dibromo-1,1,2-trifluoroethane, 1,2-dibromohexafluoropropane, 1,2-dibromotetrafluoroethane, 1,1-difluorotetrachloroethane, 1,2-difluorotetrachloroethane, heptafluoro-2,3,3-trichlorobutane, 1,1,1,3-tetrachlorotetrafluoropropane, 1,1,1-trichloropentafluoropropane, 1,1,1-trichlorotrifluoroethane, and polychlorotrifluoroethylene; ester solvents, such as methyl formate, ethyl formate, methyl acetate, ethyl acetate, propyl acetate, methyl propionate, γ-butyrolactone, and propylene carbonate; nitrile solvents, such as acetonitrile and propionitrile; aromatic solvents, such as benzene, chlorobenzene, toluene, dichlorobenzene, fluorobenzene, and nitrobenzene; ether solvents, such as monoglyme, diglyme, diethylether, dipropylether, and tetrahydrofuran; N,N-dimethyl formamide (DMF), dimethylsulfoxide (DMSO), water, nitromethane, N,N-diethylformamide, N,N-dimethylacetamide, 1-methyl-2-pyrrolidone, 1,3-dimethyl-2-imidazolidinone (DMI), tetramethylurea, 1,3-dimethylpropyleneurea, and hexamethylphosphoramide (HMPA). These solvents can be used alone or in a combination of two or more.

Examples of preferred solvents include acetonitrile (MeCN), dichloromethane, ethyl acetate (AcOEt), and monoglyme.

The amount of solvent used in reaction step A is preferably in the range of 0.1 to 50 parts by mass, and more preferably in the range of 0.5 to 20 parts by mass, per part by mass of the organic sulfur compound ($s^s$) as the starting compound.

The composition (α) containing the fluorinated organic compound ($p^f$) can be obtained by the fluorination in reaction step A.

The composition (α) may contain one or more other substances in addition to a fluorinated organic compound ($p^f$) and a sulfur-containing substance ($b^s$). Examples of such other substances include substances used in the production of a fluorinated organic compound (e.g., catalysts and bases), byproducts, etc.

The liquid containing a fluorinated organic compound ($p^f$), which is obtained in reaction step A, can be directly subjected to post-treatment step B.

Post-treatment step B

In post-treatment step B, the composition (α) containing a crude fluorinated organic compound ($p^f$) obtained in reaction step A is reacted with a nucleophile at a temperature of 40°C or higher. This step reduces the content of the sulfur-containing substance ($b^s$) in the crude fluorinated organic compound.

Examples of the nucleophile include anion-source compounds. The anion-source compounds can be compounds capable of providing an anion in post-treatment step B. Examples of such anion source compounds include carbanion-source compounds, halogen ion-source compounds, and basic compounds. Anion-source compounds can be used alone or in a combination of two or more.

Examples of the carbanion-source compounds include NaCN, KCN, CuCN, $Zn(CN)_2$, n-BuLi, sec-BuLi, t-BuLi, PhLi, EtMgBr, EtMgCl, MeMgBr, MeMgCl, $Et_2Zn$, $Me_2Zn$, $Ph_2Zn$, PhMgBr, and PhMgCl.

Examples of the halogen ion-source compound include sodium chloride, potassium chloride, lithium chloride, hydrochloric acid, sodium bromide, potassium bromide, lithium bromide, hydrobromic acid, sodium fluoride, potassium fluoride, lithium fluoride, hydrofluoric acid, sodium iodide, potassium iodide, lithium iodide, hydroiodic acid, ammonium chloride, ammonium bromide, ammonium iodide, and ammonium fluoride.

Examples of basic compounds include at least one member selected from the group consisting of (1) acetates, carbonates, hydrogen carbonates, sulfates, sulfites, phosphates, hydrogen phosphates, alkoxide salts, hydroxide salts, hydride salts, thiosulfates, or amide salts of alkali or alkaline earth metals; (2) alkali metals; and (3) amines.

Examples of acetates include sodium acetate, potassium acetate, lithium acetate, rubidium acetate, cesium acetate, magnesium acetate, calcium acetate, and barium acetate.

Examples of carbonates include sodium carbonate, potassium carbonate, lithium carbonate, rubidium carbonate, cesium carbonate, magnesium carbonate, calcium carbonate, and barium carbonate.

Examples of hydrogen carbonates include sodium hydrogen carbonate, potassium hydrogen carbonate, lithium hydrogen carbonate, rubidium hydrogen carbonate, cesium hydrogen carbonate, magnesium hydrogen carbonate, calcium hydrogen carbonate, and barium hydrogen carbonate.

Examples of sulfates include sodium sulfate, potassium sulfate, lithium sulfate, rubidium sulfate, cesium sulfate, magnesium sulfate, calcium sulfate, and barium sulfate.

Examples of sulfites include sodium sulfite, potassium sulfite, lithium sulfite, rubidium sulfite, cesium sulfite, magnesium sulfite, calcium sulfite, and barium sulfite.

Examples of phosphates include trisodium phosphate, tripotassium phosphate, trilithium phosphate, trirubidium phosphate, tricesium phosphate, trimagnesium phosphate, tricalcium phosphate, and tribarium phosphate.

Examples of hydrogen phosphates include sodium hydrogen phosphate, potassium hydrogen phosphate, lithium hydrogen phosphate, rubidium hydrogen phosphate, cesium hydrogen phosphate, magnesium hydrogen phosphate, calcium hydrogen phosphate, and barium hydrogen phosphate.

Examples of alkoxide salts include sodium methoxide, sodium ethoxide, sodium butoxide, potassium methoxide, potassium ethoxide, potassium butoxide, lithium methoxide, and lithium ethoxide.

Examples of hydroxide salts include sodium hydroxide, potassium hydroxide, lithium hydroxide, rubidium hydroxide, cesium hydroxide, magnesium hydroxide, calcium hydroxide, and barium hydroxide.

Examples of hydride salts include sodium hydride, potassium hydride, lithium hydride, and calcium hydride.

Examples of thiosulfates include sodium thiosulfate, potassium thiosulfate, lithium thiosulfate, rubidium thiosulfate, cesium thiosulfate, magnesium thiosulfate, calcium thiosulfate, and barium thiosulfate.

Examples of sulfites include sodium sulfite, potassium sulfite, lithium sulfite, rubidium sulfite, cesium sulfite, magnesium sulfite, calcium sulfite, and barium sulfite.

Examples of amide salts include sodium amide, potassium amide, lithium amide, rubidium amide, cesium amide, magnesium amide, calcium amide, and barium amide.

Examples of alkali metals include sodium, potassium, and lithium.

Examples of amines include aliphatic amines, cycloaliphatic amines, aromatic amines, and heterocyclic amines. The amine can be preferably a tertiary amine. Specific examples of amines include triethylamine, tri(n-propyl)amine, tri(n-butyl)amine, diisopropylethylamine, cyclohexyldimethylamine, pyridine, lutidine, $\gamma$-collidine, N,N-dimethylaniline, N-methylpiperidine, N-methylpyrrolidine, and N-methylmorpholine.

Preferable examples of basic compounds include lithium hydroxide, potassium hydroxide, sodium hydroxide, calcium hydroxide, lithium carbonate, sodium carbonate, potassium carbonate, sodium hydrogen carbonate, potassium hydrogen carbonate, cesium carbonate, potassium sulfite ($K_2SO_3$), sodium sulfite ($Na_2SO_3$), sodium thiosulfate, potassium thiosulfate, and ammonia.

More preferably, examples of basic compounds include potassium hydroxide, sodium hydroxide, potassium hydrogen carbonate, sodium hydrogen carbonate, potassium carbonate, potassium sulfite ($K_2SO_3$), and sodium carbonate.

Examples of basic compounds include inorganic bases. Examples of inorganic bases include lithium hydroxide, potassium hydroxide, sodium hydroxide, calcium hydroxide, lithium carbonate, sodium carbonate, potassium carbonate, sodium hydrogen carbonate, potassium hydrogen carbonate, cesium carbonate, potassium sulfite ($K_2SO_3$), sodium sulfite ($Na_2SO_3$), sodium thiosulfate, potassium thiosulfate, and ammonia.

Examples of basic compounds include compounds represented by the formula: $M_mX_n$ (where M is a cation, X is OH, CN, or $CO_3$, m is the valence of X, and n is the valence of M).

In the formula: $M_mX_n$, M may be, for example, a metal cation or optionally substituted ammonium. M is preferably an alkali metal, an alkaline earth metal, or quaternary ammonium.

M is more preferably at least one metal cation selected from the group consisting of sodium, lithium, and potassium, and is still more preferably at least one metal cation selected from the group consisting of sodium and potassium.

Examples of the quaternary ammonium include tetraalkyl ammonium (e.g., tetramethyl ammonium, tetraethyl ammonium, tetrapropyl ammonium, tetrabutyl ammonium, and hexadecyl triethyl ammonium), and benzyl trialkyl ammonium (preferably benzyl tri-$C_1$-$C_4$ alkyl ammonium) .

The amount of the nucleophile in post-treatment step B can be, for example, within the range of 0.5 to 100 moles, preferably within the range of 1 to 70 moles, and more preferably within the range of 2 to 50 moles, per mole of the organic sulfur compound ($S^S$).

The treatment temperature in post-treatment step B can preferably be 50°C or higher, more preferably 60°C or higher.

The treatment temperature in post-treatment step B can be, for example, 200°C or less, preferably 170°C or less, and more preferably 150°C or less.

The lower limit and the upper limit can be appropriately combined.

The treatment temperature in post-treatment step B can be, for example, in the range of 40°C to 200°C, in the range of 40°C to 170°C, or in the range of 60°C to 170°C, preferably in the range of 50°C to 170°C, and more preferably in the range of 60°C to 150°C.

When the treatment temperature in post-treatment step B is within the range described above, the effect of reducing the sulfur-containing substance ($b^s$) is great.

The treatment time of post-treatment step B can be, for example, 0.5 hours or more, preferably 1 hour or more, and more preferably 3 hours or more.

The treatment time of post-treatment step B can be, for example, 72 hours or less, preferably 48 hours or less, and more preferably 24 hours or less.

The lower limit and the upper limit can be appropriately combined.

The treatment time of post-treatment step B can be, for example, in the range of 0.5 hours to 72 hours, in the range of 0.5 hours to 48 hours, or in the range of 0.5 hours to 24 hours, and preferably in the range of 1 hour to 48 hours.

Post-treatment step B can be preferably carried out in the presence of water. For example, the composition

($\alpha$) obtained in reaction step A (for example, a reaction liquid obtained in reaction step A) is mixed with water in which a nucleophile has been dissolved or dispersed, and the resulting mixture is subjected to a desired temperature condition to thereby carry out post-treatment step B. The amount of water is not particularly limited as long as the reaction with the nucleophile proceeds.

Post-treatment step B can be preferably carried out in an organic solvent. For example, by mixing the composition ($\alpha$) obtained in reaction step A (for example, the reaction liquid obtained in reaction step A) with water in which a nucleophile has been dissolved or dispersed and an organic solvent and subjecting the resulting mixture to a desired temperature condition, post-treatment step B can be performed. When the composition ($\alpha$) obtained in reaction step A contains an organic solvent, post-treatment step B can be performed without adding any organic solvent, but adding an organic solvent is preferable.

Post-treatment step B can be more preferably carried out in the presence of water and an organic solvent. For example, post-treatment step B can be carried out by mixing the composition ($\alpha$) obtained in reaction step A (for example, a reaction liquid obtained in reaction step A) with water in which a nucleophile has been dissolved or dispersed and an organic solvent and subjecting the resulting mixture to desired temperature conditions. In post-treatment step B, stirring is preferably performed to promote the reaction.

When post-treatment step B is performed in an organic solvent, the organic solvent may be an organic solvent WS, an organic solvent WP, or a solvent containing an organic solvent WS and an organic solvent WP. The amount of the organic solvent used is not particularly limited as long as post-treatment step B can be carried out. The amount of the organic solvent may be any amount as long as a crude fluorinated organic compound ($p^f$) is dissolved at a temperature of 40°C or higher.

The organic solvent is preferably an organic solvent WS having water solubility of at least 10 g/100 g of water at 20°C. By using such an organic solvent, the effect of reducing the content of the sulfur-containing substance ($b^s$) is further enhanced.

The solubility to water is the maximum amount of organic solvent that can be dissolved in 100 g of water at a water temperature of 20°C. Table 1 shows the solubility of some organic solvents in water at a water temperature of 20°C.

Table 1

| Solvent | Molecular formula | Solubility (g/100 g) |
|---|---|---|
| Hexane | $C_6H_{22}$ | 0.0013 |
| Toluene | $C_7H_8$ | 0.05 |
| Chloroform | $CHCl_3$ | 0.795 |
| Dichloromethane | $CH_2Cl_2$ | 1.32 |
| Diethyl ether | $C_4H_{10}O$ | 7.5 |
| 2-Methyltetrahydrofuran | $C_5H_{10}O$ | 14 |
| Tetrahydrofuran | $C_4H_8O$ | 30 |
| Dioxane | $C_4H_8O_2$ | Infinite |
| Acetone | $C_3F_6O$ | Infinite |
| Acetonitrile | $C_2H_3N$ | Infinite |
| Monoglyme | $C_4H_{10}O_2$ | Infinite |

The solubility of organic solvent WS in water can be, for example, 10 or more, preferably 15 or more, and more preferably 20 or more. The solubility of organic solvent WS in water is more preferably infinite. Organic solvents WS can be appropriately combined and used in combination.

The solubility of organic solvent WS in water can be, for example, 10 to infinity, preferably 15 to infinity, and more preferably 20 to infinity.

Preferable examples of organic solvent WS include monomethyl, acetonitrile, acetone, dioxane (preferably 1,4-dioxane), N-methylpyrrolidone (abbreviated name: NMP), diethylene glycol, 3-butene, 2-butanol, tetrahydrofuran (abbreviated name: THF), 2-methyl THF, N,N-dimethylformamide (abbreviated name: DMF), dimethylacetamide (abbreviated name: DMAc) and the like; more preferable are monomethyl, acetonitrile, acetone, dioxane (preferably 1,4-dioxane), and THF.

The organic solvent can be a mixed solvent of organic solvent WS and organic solvent WP whose water-solubility is less than 10 g/100 g of water (20°C).

The solubility of organic solvent WP in water can be, for example, less than 10, preferably 5 or less, and more preferably 3 or less.

The solubility of organic solvent WP in water can be, for example, 0.01 or more.

The lower limit and the upper limit can be appropriately combined.

The solubility of organic solvent WP in water can be, for example, in the range of 0.001 to less than 10, preferably in the range of 0.001 to 5, and more preferably in the range of 0.001 to 3.

Examples of the organic solvent WP include toluene, dichloromethane, hexane, heptane, xylene, benzene, diethyl ether, methyl tert-butyl ether (abbreviated name: MTBE), ethyl acetate, chloroform, and cyclopentyl methyl ether (CPME), and more preferably toluene, dichloromethane, and heptane.

When a mixture of organic solvents WS and WP is used, the mass of organic solvent WS in the mixture can be, for example, 0.1 g or more, and preferably 0.5 g or more, per gram of mass of the organic solvent WP.

The mass of organic solvent WS in the mixed solvent can be, for example, 20 g or less, and preferably 10 g or less, per gram of mass of the organic solvent WP in the mixed solvent.

The lower limit and the upper limit can be appropriately combined.

The mass of organic solvent WS in the mixed solvent can be, for example, in the range of 0.1 g to 20 g, preferably in the range of 0.5 g to 10 g, per gram of mass of the organic solvent WP in the mixed solvent.

The reaction liquid obtained in post-treatment step B is subjected, as desired, to a conventional method, such as liquid separation, extraction, dissolution, concentration, precipitation, dehydration, adsorption, distillation, rectification, or chromatography, or a combination of these methods, to thereby obtain a crude fluorinated organic compound ($p^f$) with a reduced content of sulfur-containing substance ($b^s$). The crude product can be obtained, for example, by a method comprising concentrating (1) a reaction liquid obtained by reacting the composition ($\alpha$) with a nucleophile in step B or (2) an organic phase obtained by separating the reaction liquid to a concentration to obtain a solid, then dissolving the solid in a solvent to obtain a solution, and then precipitating a fluorinated organic compound ($p^f$) (a recrystallization method); or a method comprising separating the reaction liquid to obtain an organic layer and distilling off the solvent of the organic layer under reduced pressure. The sulfur content of the fluorinated organic compound ($p^f$) can be further reduced by conducting these additional treatments.

The step of obtaining the solid can be carried out by a known method, such as a method of concentrating the reaction liquid or the organic phase under reduced pressure.

The step of obtaining the solution can be performed by a known method, such as a method of dissolving the solid in an excess amount of a solvent capable of dissolving the solid. The solvent used in the step of obtaining the solution is not particularly limited as long as the solid can be dissolved. Examples include heptane, hexane, methanol, and ethanol.

The step of precipitating the fluorinated organic compound ($p^f$) can be carried out by, for example, a method comprising adding a poor solvent to the solution to precipitate a fluorinated organic compound ($p^f$). The poor solvent is not particularly limited as long as the fluorinated organic compound ($p^f$) can be precipitated.

In the recrystallization method, a treatment liquid obtained by treating the reaction liquid or the organic phase with silica gel, celite, activated carbon, or the like, may be subjected to the subsequent concentration step.

In the recrystallization method, a treatment liquid obtained by treating the solution with silica gel, celite, activated carbon, of the like, may be subjected to the subsequent step of precipitating the fluorinated organic compound ($p^f$).

The treatment with silica gel or the like described above can be carried out by any known appropriate method. Examples of methods include a method comprising adding silica gel, celite, activated carbon, or the like to the reaction solution or organic phase described above, subjecting the resulting mixture to solid-liquid separation (e.g. filtration) to obtain a separation liquid (e.g. filtrate), and a method comprising subjecting the reaction liquid or organic phase to silica gel, celite, activated carbon, or the like to column chromatography with silica gel, celite, activated carbon, or the like to obtain a distillate.

## Composition ($\beta$)

Since the composition ($\beta$) has a reduced content of sulfur-containing substance ($b^s$), the composition ($\beta$) is useful as a source of supplying a fluorinated organic compound ($p^f$).

The composition ($\beta$) can be preferably produced by the method for producing a fluorinated organic compound of the present disclosure.

The lower limit of the fluorinated organic compound ($p^f$) content in the composition ($\beta$) is preferably 80 mass%, more preferably 90 mass%, and still more preferably 95 mass%.

The upper limit of the fluorinated organic compound ($p^f$) content in the composition ($\beta$) is preferably 99.9 mass%, more preferably 99.5 mass%, and still more preferably 99 mass%.

The lower limit and the upper limit can be appropriately combined.

The content of the fluorinated organic compound in the composition (β) is preferably in the range of 80 to 99.9 mass%, more preferably in the range of 90 to 99.5 mass%, and still more preferably in the range of 95 to 99 mass%.

The upper limit of the content of sulfur-containing substance ($b^s$) in the composition (β), in terms of sulfur content, can be preferably 10000 ppm by mass, more preferably 7000 ppm by mass, and still more preferably 6000 ppm by mass, with respect to the mass of composition (β).

The lower limit of the content of the sulfur-containing substance in the composition (β), in terms of sulfur content, can be preferably 0.01 ppm by mass, preferably 0.1 ppm by mass, and still more preferably 0.5 ppm by mass, with respect to the mass of the composition (β).

The lower limit and the upper limit can be appropriately combined.

The content of the sulfur-containing substance in the composition (β), in terms of sulfur content, can be preferably in the range of 0.01 to 10000 ppm by mass, more preferably in the range of 0.1 to 7000 ppm by mass, and still more preferably in the range of 0.5 to 6000 ppm by mass, with respect to the mass of the composition (β).

The content of the sulfur-containing substance ($b^s$) in the composition (β), in terms of sulfur content, can be preferably such that the mass ratio of the content of sulfur-containing substance ($b^s$) to the content of fluorinated organic compound ($p^f$) in the composition (β) is 1/10 or less, more preferably 1/50 or less, and even more preferably 1/100 or less.

The mass ratio of the content of sulfur-containing substance ($b^s$), in terms of sulfur content, to the content of fluorinated organic compound ($p^f$) in the composition (β) can be preferably 1/1000000 or more, more preferably 1/500000 or more, and still more preferably 1/300000 or more.

The lower limit and the upper limit can be appropriately combined.

The mass ratio of the content of sulfur-containing substance ($b^s$), in terms of sulfur content, to the content of fluorinated organic compound ($p^f$) can be preferably in the range of 1/1000000 to 1/10, more preferably in the range of 1/500000 to 1/50, and still more preferably in the range of 1/300000 to 1/100.

In the present disclosure, the method for analyzing the sulfur content includes, for example, methods of oxidative decomposition and ultraviolet absorption. Specifically, the methods described in the Examples can be used.

## Examples

Embodiments of the present disclosure are described in more detail with reference to Examples and the like. However, the present disclosure is not limited to these. Each operation in the Examples and the like was performed in a room temperature environment without controlling the temperature, unless otherwise specified. In the Examples and the like, $Et_3N$ represents triethyl amine, Me represents methyl, and AcOEt represents ethyl acetate.

### Method for measuring sulfur content

The sulfur content in the Examples and the like was measured by the following method.

Measurement apparatus:

Trace sulfur analyzer (ultraviolet fluorescence method)
(Mitsubishi Chemical Analytech TS-100)

Preparation of sample solution:
10 to 15 mg of an accurately weighed sample and 10 mL of acetone were placed into a glass vial to dissolve the sample. Alternatively, 10 to 15 mg of the accurately weighed sample can be used as it is, i.e., without being dissolved, to measure the sulfur content.

Preparation of blank solution:
10 mL of acetone was placed into a glass vial.

Measurement:

(1) 100 μL of a blank solution was placed in a quartz boat that had been baked empty twice. Acetone was distilled off.
(2) The quartz boat was set in a sample chamber and measurement was performed.
(3) Each sample solution was also measured in the same manner by the above procedures (1) and (2). Note that when the sample is used without being dissolved in acetone, the sample is directly placed in a quartz boat and measurement can be performed in the same manner by the above procedures (1) and (2).

Sulfur content calculation:
Based on the peak areas of the blank solution and the sample solutions obtained by the measurement, the sulfur content was calculated according to the following formula:

$$S = ((A-B) - b)/a \times V/Vt/W \times 1000$$

wherein

S: sulfur content of the sample (mass fraction: ppm)
W: mass of the sample (mg)
V: amount of solvent used for dissolving the sample (mL)
Vt: volume used for measurement (μL)
A: area value of the sample
B: area value of the blank
a: slope of calibration curve
b: Y-intercept of calibration curve

Calibration curves were prepared by using dibutyl disulfide as a specimen.

Example 1

After 3,5,2'-trifluoro-4"-propyl[1,1';4',1"]terphenyl-4-carbothioic acid 3,4,5-trifluorophenyl ester (185 g, 0.35 mol) was dissolved in MeCN (143 g) and monoglyme (103 g) and was subjected to a fluorination reaction with $IF_5$-$Et_3N$-3HF (150.9 g, 0.39 mol), an aqueous solution of toluene (206 g), KOH (176.9 g, 3.16 mol), and $K_2SO_3$ (187.1 g, 1.18 mol) in $H_2O$ (925.1 g) was added to the obtained solution and mixed. The resulting mixture was stirred at 80°C for 24 hours. The organic layer obtained by separating the liquid after stirring was filtered, and then the solvent was distilled off under reduced pressure to obtain a crude 4-[difluoro(3,4,5-trifluorophenoxy)methyl]-3,5,2'-trifluoro-4"-propyl[1,1';4',1"]terphenyl (182 g; desired fluorinated compound content: 173 g). Table 2 shows the sulfur content of the obtained crude product.

Example 2

Using the same molar amounts and the same procedure as in Example 1, a crude 4-[difluoro(3,4,5-trifluorophenoxy)methyl]-3,5,2'-trifluoro-4"-pentyl[1,1';4',1"]terphenyl (186 g; desired fluorinated compound content: 182 g) was obtained from 3,5,2'-trifluoro-4"-pentyl[1,1';4',1"]terphenyl-4-carbothioic acid 3,4,5-pentafluorophenyl ester.

Example 3

After 4-chloro-2,6-difluorothiobenzoic acid 3,4,5-trifluorophenyl ester (255.6 g, 0.75 mol) was dissolved in MeCN (364.2 g) and was subjected to a fluorination reaction with $IF_5$-$Et_3N$-3HF (231.3 g, 0.60 mol), an aqueous solution of toluene (322.5 g), KOH (271.0 g, 4.84 mol), and $K_2SO_3$ (286.6 g, 1.81 mol) dissolved in water (1417.6 g) was added to the obtained solution and mixed. The resulting mixture was stirred at 85°C for 13 hours. After the stirred liquid was subjected to liquid separation, the organic layer was filtered and the solvent was then distilled off under reduced pressure to obtain a crude 5-[(4-chloro-2,6-difluorophenyl)difluoromethoxy]1,2,3-trifluorobenzene (266 g; target fluorinated compound content: 245 g). Table 2 shows the sulfur content of the obtained crude product.

Table 2

| | Sulfur content (ppm) |
|---|---|
| Example 1 | 5670 |
| Example 2 | 4673 |
| Example 3 | 3208 |

Examples 4 and 5

The procedure was performed in the same manner as in Example 1 to obtain a crude 4-[difluoro(3,4,5-trifluorophenoxy)methyl]3,5-difluoro-4'-propylbiphenyl from 3,5-difluoro-4'-propylbiphenyl-4-carbothioic acid 3,4,5-trifluorophenyl ester. This crude product (500 mg, sulfur content: 5321 ppm) was dissolved in each solvent shown in Table 3, and additives shown in Table 3 were added. Heating and stirring were then performed under the conditions shown in Table 3. After the resulting mixture was allowed to cool, water and heptane were added, and the organic substance was extracted. The obtained organic layer was concentrated under reduced pressure and evaporated to dryness to obtain white crystals (495 mg; desired fluorinated compound content: 485 mg). The sulfur content of the obtained white crystals was analyzed. Table 3 shows the results.

Table 3

| | Solvent | Additive | Temperature (°C) | Heating time (hours) | Sulfur content (ppm) |
|---|---|---|---|---|---|
| Example 4 | Acetone (2 g), Water (1 g) | NaCN (200 mg) | 56 | 0.5 | 21.8 |
| Example 5 | 1,4-Dioxane 2 g), Water (1 g) | NaOH (200 mg) | 101 | 4.0 | 4.9 |

Example 6

After diphenylmethanethione (5 g, 25.3 mmol) was dissolved in $CH_2Cl_2$ (100 g) and subjected to a fluorination reaction with $IF_5$-$Et_3N$-3HF (11.6 g, 30.3 mmol), an aqueous solution of MeCN (120 g), KOH (13.6 g, 242 mmol) and $K_2SO_3$ (19.1 g, 121.2 mmol) in water (65 g) was added to the obtained solution. The resulting mixture was stirred at 40°C for 24 hours. After the resulting liquid was subjected to liquid separation, the obtained organic layer was filtered and the solvent was distilled off under reduced pressure to obtain a crude difluorodiphenylmethane (4.4 g; desired fluorinated compound content: 3.74 g). Table 4 shows the sulfur content of the obtained crude product.

Example 7

After methyl N-butyl-N-(1-oxopropyl)carbamodithioate (0.5 g, 2.28 mmol) was dissolved in dichloromethane (10 g) and was subjected to a fluorination reaction with $IF_5$-$Et_3N$-3HF (0.87 g, 2.28 mmol), an aqueous solution of MeCN (40 g), KOH (6.5 g, 115.2 mmol), and $K_2SO_3$ (9.1 g, 57.6 mmol) dissolved in water (30 g) was added to the obtained solution. The resulting mixture was stirred at 75 to 80°C for 3 hours. After the obtained liquid was subjected to liquid separation, the obtained organic layer was filtered, and the solvent was distilled off under reduced pressure to obtain a crude N-butyl-N-trifluoromethylpropionamide (300 mg; desired fluorinated compound content: 245 mg). Table 4 shows the sulfur content of the obtained crude product.

Example 8

Using the same molar amounts and procedure as in Example 7, a crude 1-trifluoromethoxydecane and 1-(difluoro(methylthio)methoxy)decane (490 mg; desired fluorinated compound content: 440 mg) was obtained from O-decyl S-methyl carbonodithioate. Table 4 shows the sulfur content of the obtained crude product.

Example 9

The procedure was performed in the same manner as in Example 7 except that AcoEt was used in place of dichloromethane as a solvent for reaction to thereby obtain a crude 4-pentyl-4'-trifluoromethoxybicyclohexyl (378 mg; desired fluorinated compound content: 310 mg) from S-methyl O-(4'-pentylbicyclohexyl-4-yl)carbonodithioate. Table 4 shows the sulfur content of the obtained crude product.

Example 10

Using the same molar amounts and procedure as in Example 6, a crude difluorodiphenylmethane (5.2 g; desired fluorinated compound content: 4.7 g) was obtained from 2,2-diphenyl-[1,3]dithiolane. Table 4 shows the sulfur content of the obtained crude product.

Example 11

The procedure was performed in the same manner as in Example 7 except that that NBS-pyridine-9HF was used in place of $IF_5$-$Et_3N$-3HF as a fluorinating agent to thereby obtain a crude 4-pentyl-4'-trifluoromethoxybicyclohexyl (650 mg; desired fluorinated compound content: 550 mg) from 4'-(difluoro(methylthio)methoxy)-4-pentylbicyclohexyl. Table 4 shows the sulfur content of the obtained crude product.

Table 4

| | Sulfur content (ppm) |
|---|---|
| Example 6 | 5876 |
| Example 7 | 269 |
| Example 8 | 1341 |
| Example 9 | 1567 |
| Example 10 | 4724 |
| Example 11 | 1788 |

Example 12

Silica gel (20 g) was added to the organic layer after liquid separation and filtration obtained in Example 1. The resulting mixture was stirred at room temperature for 30 minutes and then filtered. The obtained organic layer was concentrated under reduced pressure to obtain a crude product (158 g; desired fluorinated compound content: 156 g, sulfur content: 99 ppm). Further, heptane (555 g) was added to the obtained crude product and the resulting mixture was subjected to a crystallization step to obtain 4-[difluoro(3,4,5-trifluorophenoxy)methyl]-3,5,2'-trifluoro-4"-propyl[1,1';4',1"]terphenyl (126 g, sulfur content: 23 ppm).

Example 13

The organic layer after liquid separation and filtration obtained in Example 1 was concentrated under reduced pressure to obtain a solid. The solid was dissolved in heptane (555 g) and purified with a silica gel column. After heptane was removed from the obtained distillate under reduced pressure to concentrate the distillate, a crystallization step was performed using ethanol (516 g) to thereby obtain 4-[difluoro(3,4,5-trifluorophenoxy)methyl]-3,5,2'-trifluoro-4"-propyl[1,1';4',1"]terphenyl (172 g, sulfur content: 1 ppm).

Example 14

After 4-chloro-2,6-difluorothiobenzoic acid 3,4,5-trifluorophenyl ester (50 mg, 0.147 mmol) was dissolved in dichloromethane (1 g) and was subjected to a fluorination reaction with $BrF_3$-1.5($KHF_2$) (74.9 mg, 0.295 mmol), an aqueous solution of MeCN (4 g), KOH (99.1 mg, 1.770 mmol) and $K_2SO_3$ (186.4 mg, 1.180 mmol) in water (3 g) was added and mixed. The resulting mixture was stirred at 60°C for 3 hours. The liquid after stirring was subjected to liquid separation and the resulting organic layer was filtered. The solvent was then distilled off under reduced pressure to obtain a crude 5-[(4-chloro-2,6-difluorophenyl)difluoromethoxy]1,2,3-trifluorobenzene (52.2 mg; desired fluorinated compound content: 48 mg). The sulfur content of the obtained crude product was 256 ppm.

## Claims

**1.** A method for producing a fluorinated organic compound ($p^f$), comprising:

[A] a reaction step A of fluorinating an organic sulfur compound ($s^s$) with a fluorinating agent to obtain a composition ($\alpha$) containing a fluorinated organic compound ($p^f$); and
[B] a post-treatment step B of reacting the composition ($\alpha$) with a nucleophile at a temperature of 40°C or higher.

**2.** The production method according to claim 1, wherein the reaction in the post-treatment step B is performed in the presence of water.

3. The production method according to claim 1 or 2, wherein the fluorinating agent comprises a fluoride ion.

4. The production method according to any one of claims 1 to 3, wherein the fluorinating agent comprises an oxidant and a fluoride ion.

5. The production method according to claim 4, wherein the oxidant is at least one member selected from the group consisting of halogen oxidants and ammonium salt oxidants.

6. The production method according to claim 4 or 5, wherein the oxidant comprises at least one member selected from the group consisting of
$IF_5$,

bromine trifluoride,
compounds represented by formula (3a): $X^2$-Z (3a)

wherein $X^2$ is a halogen atom,
Z is a halogen atom or $NZ^1Z^2$,
$Z^1$ and $Z^2$ are each independently an organic group, or
$Z^1$ and $Z^2$ bind together to form a ring, and compounds represented by formula (3a')

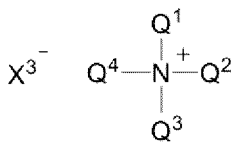 (3a')

wherein $X^3$ is a halogen atom, $ClO_4$, or $NO_3$,
$Q^1$ to $Q^4$ are each independently H or an organic group, or any two of $Q^1$ to $Q^4$ may bind together to form a ring.

7. The production method according to claim 6, wherein

$X^2$ is an iodine atom, a bromine atom, or a chlorine atom,
Z is an iodine atom, a bromine atom, a chlorine atom, or $NZ^1Z^2$, and
$X^3$ is $ClO_4$ or $NO_3$.

8. The production method according to any one of claims 1 to 6, wherein the fluorinating agent comprises $IF_5$, a fluoride ion, and an amine.

9. The production method according to any one of claims 1 to 6, wherein the fluorinating agent comprises bromine trifluoride and a fluoride ion.

10. The production method according to any one of claims 1 to 9, wherein the nucleophile is an anion-source compound.

11. The production method according to claim 10, wherein the anion-source compound is at least one member selected from the group consisting of a carbanion-source compound, a halogen ion-source compound, and a basic compound.

12. The production method according to claim 11, wherein the basic compound is an inorganic base.

13. The production method according to claim 11, wherein the basic compound is a compound represented by formula: $M_mX_n$ wherein

M is a cation,
X is OH, CN, or $CO_3$,
m is the valence of X, and
n is the valence of M.

14. The production method according to claim 13, wherein M is a metal cation or an optionally substituted ammonium.

**15.** The production method according to claim 13, wherein M is an alkali metal, an alkaline earth metal, or a quaternary ammonium.

**16.** The production method according to any one of claims 11 to 15, wherein the basic compound is one or two compounds selected from the group consisting of KOH and NaOH.

**17.** The production method according to any one of claims 1 to 16, wherein the temperature in the post-treatment step B is 50°C or higher.

**18.** The production method according to any one of claims 1 to 17, wherein the post-treatment step B is performed for 0.5 hours or more.

**19.** The production method according to any one of claims 1 to 18, wherein the post-treatment step B is performed in an organic solvent WS having a solubility in water of at least 10 g/100 g of water at 20°C.

**20.** The production method according to claim 19, wherein

the post-treatment step B is performed in a mixed solvent containing the organic solvent WS and an organic solvent WP whose solubility in water is less than 10 g/100 g of water at 20°C, and
the mass of the organic solvent WS relative to the mass of the organic solvent WP is 0.1 g/g or more.

**21.** The production method according to any one of claims 1 to 20, wherein (1) a reaction liquid obtained by reacting the composition ($\alpha$) with a nucleophile or (2) an organic phase obtained by separating the reaction liquid is subjected to at least one treatment selected from the group consisting of crystallization, adsorption, and distillation.

**22.** The production method according to claim 22, wherein an adsorbent used for the adsorption is silica or alumina.

**23.** A composition ($\beta$) comprising a fluorinated organic compound ($p^f$) and a sulfur-containing substance ($b^s$) with the proviso that the fluorinated organic compound ($p^f$) is excluded from the sulfur-containing substance ($b^s$), wherein the content of the sulfur-containing substance ($b^s$) is 10000 ppm by mass or less in terms of sulfur.

**24.** The composition ($\beta$) according to claim 23, wherein the content of the fluorinated organic compound ($p^f$) is 80 mass% or more.

**25.** The composition ($\beta$) according to claim 23 or 24,
wherein the mass ratio of the content of the sulfur-containing substance ($b^s$) in terms of sulfur to the content of the fluorinated organic compound ($p^f$) is 1/10 or less.

**26.** The composition ($\beta$) according to any one of claims 23 to 25, wherein the content of the sulfur-containing substance ($b^s$) is 7000 ppm by mass or less in terms of sulfur.

**27.** The composition ($\beta$) according to any one of claims 23 to 26, wherein the content of the sulfur-containing substance ($b^s$) is 6000 ppm by mass or less in terms of sulfur.

**28.** The composition ($\beta$) according to any one of claims 23 to 27, wherein the content of the sulfur-containing substance ($b^s$) is 0.01 ppm by mass or more in terms of sulfur.

**29.** The composition ($\beta$) according to any one of claims 23 to 28, wherein the content of the sulfur-containing substance ($b^s$) is 0.1 ppm by mass or more in terms of sulfur.

## INTERNATIONAL SEARCH REPORT

International application No.
**PCT/JP2022/013427**

### A. CLASSIFICATION OF SUBJECT MATTER

***C07C 17/093***(2006.01)i; ***C07C 17/361***(2006.01)i; ***C07C 22/08***(2006.01)i; ***C07C 41/22***(2006.01)i; ***C07C 43/12***(2006.01)i; ***C07C 43/192***(2006.01)i; ***C07C 43/225***(2006.01)i; ***C07C 231/12***(2006.01)i; ***C07C 233/13***(2006.01)i; ***C07C 319/20***(2006.01)i; ***C07C 323/12***(2006.01)i; *C07B 39/00*(2006.01)n
FI: C07C17/093; C07C17/361; C07C22/08; C07C41/22; C07C43/12; C07C43/192; C07C43/225 A; C07C43/225 D; C07C231/12; C07C233/13; C07C319/20; C07C323/12; C07B39/00 B

According to International Patent Classification (IPC) or to both national classification and IPC

### B. FIELDS SEARCHED

Minimum documentation searched (classification system followed by classification symbols)

C07C17/093; C07C17/361; C07C22/08; C07C41/22; C07C43/12; C07C43/192; C07C43/225; C07C231/12; C07C233/13; C07C319/20; C07C323/12; C07B39/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 01/96263 A1 (DAIKIN INDUSTRIES, LTD) 20 December 2001 (2001-12-20) p. 16, line 28 to p. 17, line 2, p. 23, line 23 to p. 24, line 1, pp. 40-45, claims | 1-29 |
| A | WO 2015/141811 A1 (DAIKIN INDUSTRIES, LTD) 24 September 2015 (2015-09-24) examples, claims | 1-29 |
| A | JP 2006-151894 A (DAIKIN INDUSTRIES, LTD) 15 June 2006 (2006-06-15) claims, examples | 1-29 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 May 2022** | **24 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/013427**

| Patent document cited in search report | Publication date (day/month/year) | Patent family member(s) | Publication date (day/month/year) |
|---|---|---|---|
| WO 01/96263 A1 | 20 December 2001 | JP 4892808 B2 | |
| | | US 2003/0176747 A1 paragraphs [0131], [0132], tables 1-6, claims | |
| | | US 2004/0245505 A1 | |
| | | EP 1304316 A1 | |
| | | KR 10-0530923 B1 | |
| | | CN 1436159 A | |
| WO 2015/141811 A1 | 24 September 2015 | JP 2015-193609 A | |
| | | EP 3121160 A1 examples, claims | |
| | | CN 106132915 A | |
| | | KR 10-2016-0131101 A | |
| | | CN 110790641 A | |
| JP 2006-151894 A | 15 June 2006 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200196263 A
- WO 2015141811 A